# EUROPEAN PATENT APPLICATION

(11) **EP 3 747 932 A1**
(43) Date of publication of application: **09.12.2020**
(21) Application number: 19747183.2
(22) Date of filing: 23.01.2019
(51) Int. Cl.: C08G 69/10, C08G 69/16, C08G 69/08, C08G 69/48, A01N 37/46, A01N 47/44, A01N 43/38, A01N 43/54, A61K 31/785, A61K 31/787, A01P 1/00, A01P 3/00

(54) **BRANCHED POLYAMINO ACID BACTERIOSTATIC AGENT AND APPLICATION THEREOF**

(30) Priority: 31.01.2018 CN 201810096212; 31.01.2018 CN 201810096221; 31.01.2018 CN 201810097056
(71) Applicant: Changchun Institute of Applied Chemistry, Chinese Academy of Sciences, Changchun, Jilin 130022 (CN)
(72) Inventor: JI, Shengxiang, Jilin 130022 (CN); LIU, Xiao, Jilin 130022 (CN); HAN, Miaomiao, Jilin 130022 (CN); LIU, Yadong, Jilin 130022 (CN); GUO, Jianwei, Jilin 130022 (CN)
(74) Representative: Witte, Weller & Partner Patentanwälte mbB
(86) International application number: PCT/CN2019/072812
(87) International publication number: WO 2019/149121

(57) **Abstract**

The present invention provides a branched poly(amino acid) antimicrobial agent, comprising a branched poly(amino acid); the branched poly(amino acid) is obtained by the homopolymerization of one amino acid unit, or is obtained by the copolymerization of two or more amino acid units; the amino acid unit has a structure shown by Formula I. The present invention uses the amino acid as raw material, is non-toxic, has no side effects, and is a green and environmentally friendly new antimicrobial agent, and accessible to the users. The branched structure of the poly(amino acid) results in that such material has many active functional groups, may be further modified, has good biocompatibility, and will not develop drug resistance during the long-term use of this antimicrobial agent.

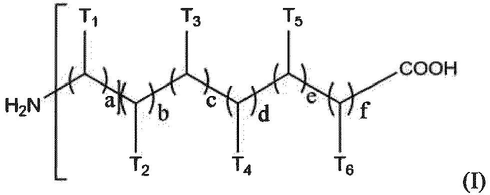

## Description

### FIELD OF THE INVENTION

The present invention relates to the technical field of antimicrobial agents, and in particular to branched poly(amino acid) antimicrobial agents and use thereof.

### BACKGROUND OF THE INVENTION

The development of antibiotics has saved the lives of hundreds of millions of people and prevented humans suffering from the pain of bacterial infection. It can be considered as a revolution in medicine. However, with the abuse of small-molecule antibiotics, coupled with the short life cycle of bacteria and gene transfer characteristics, various drug-resistant bacteria have emerged. The spread of pathogenic bacteria seriously endangers people's normal lives. A new study shows that if not controlled, super bacteria will kill about 10 million people every year by 2050. Drug-resistant bacteria have become a hot issue of common concern in various countries around the world, which is related to global human health, economic development, and social stability.

Compared with small-molecule antibiotics, polymeric antimicrobial agents can non-specifically bind to negatively charged bacterial membranes, and then insert into the bacterial cell membrane, causing the bacterial cell membrane to rupture so as to kill the bacteria. Thus, it is difficult for the polymeric antimicrobial agents to develop a drug resistance. Therefore, it is a major issue of significance to develop and use of an antimicrobial polymeric material with high safety, good antimicrobial effect, sustainability, and biodegradability.

Amino acids are renewable resources, mainly synthesized from biomass (starch, cellulose, etc.) as raw materials through hydrolysis and fermentation, and the global annual output reaches million tons. At present, amino acids are mainly used as food and feed additives, with low added values. How to develop new products with high added values is an urgent problem to be solved in the amino acid industry. The application of branched poly(amino acid)s in the field of antimicrobial agents has not been reported yet.

### SUMMARY OF THE INVENTION

In view of this, the technical problem to be solved by the present invention is to provide branched poly(amino acid) antimicrobial agents and use thereof. Said antimicrobial agents utilize the amino acid as raw material, and the prepared branched poly(amino acid)s have excellent antimicrobial performances.

To solve above technical problem, the present invention provides a branched poly(amino acid) antimicrobial agent, comprising a branched poly(amino acid);

The branched poly(amino acid) is obtained by the homopolymerization of one amino acid unit, or by the copolymerization of two or more amino acid units;

The amino acid unit has a structure of Formula I or salts thereof: 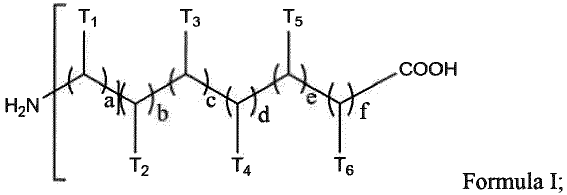 wherein,
a, b, c, d, e, and f are independently an integer of 0∼6, and 1≤a+b+c+d+e+f≤20 (e.g., a+b+c+d+e+f = 1, 2, 3, 4, 5, 6, 7, 8, 9, 10, 11, 12, 13, 14, 15, 16, 17, 18, 19, or 20), preferably a+b+c+d+e+f≤10;
T₁, T₂, T₃, T₄, T₅, and T₆ are independently selected from the group consisting of hydrogen, hydroxyl, mercapto, amino, carboxyl, C1∼C18 (e.g., a carbon number of 1, 2, 3, 4, 5, 6, 7, 8, 9, 10, 11, 12, 13, 14, 15, 16, 17, and 18) alkyl and derivatives thereof, C6∼C30 (preferably a carbon number of 6-18) aryl and derivatives thereof, C3∼C8 (e.g., a carbon number of 3, 4, 5, 6, 7, 8) cycloalkyl and derivatives thereof, C2∼C8 (e.g., a carbon number of 2, 3, 4, 5, 6, 7, 8) alkenyl and derivatives thereof, C2∼C8 (e.g., a carbon number of 2, 3, 4, 5, 6, 7, 8) alkynyl and derivatives thereof, C1∼C8 (e.g., a carbon number of 1, 2, 3, 4, 5, 6, 7, 8) alkoxy and derivatives thereof, C1∼C8 (e.g., a carbon number of 1, 2, 3, 4, 5, 6, 7, 8) alkylthio and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof. Optionally, T₁, T₂, T₃, T₄, T₅, and T₆ are not H at the same time.

Formula I as recited above is the general structural formula of the amino acid unit. The branched poly(amino acid) may be obtained by the copolymerization of two or more amino acid units, or by the homopolymerization of one amino acid unit.

In a preferred embodiment of the present invention, the branched poly(amino acid) is obtained by the homopolymerization of one amino acid unit, wherein at least one of T₁, T₂, T₃, T₄, T₅, and T₆ of the amino acid units is selected from the group consisting of hydroxyl, amino, mercapto, carboxyl, C2∼C8 alkenyl and derivatives thereof, C2∼C8 alkynyl and derivatives thereof, C1∼C8 alkoxy and derivatives thereof, C1∼C8 alkylthio and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof.

In another preferred embodiment of the present invention, the branched poly(amino acid) is obtained by the copolymerization of two or more amino acid units, wherein at least one of T₁, T₂, T₃, T₄, T₅, and T₆ of at least one amino acid unit is selected from the group consisting of hydroxyl, amino, mercapto, carboxyl, C2∼C8 alkenyl and derivatives thereof, C2∼C8 alkynyl and derivatives thereof, C1 ∼ C8 alkoxy and derivatives thereof, C1 ∼ C8 alkylthio and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof.

The branched poly(amino acid) has a number-average molecular weight of about 500 g/mol-500,000 g/mol.

Preferably, the molecular weight of the polymer in the present invention is measured by gel-permeation chromatograph (GPC). As shown in the Examples, a specific measurement method is as follows: the molecular weight (*M*ₙ) of the polymer and the distribution thereof (PDI=*M*_{w}/*M*ₙ) are measured by Waters 2414 gel-permeation chromatograph system equipped with Waters 2414 interference refraction detector (mobile phase: 0.2M acetic acid/0.1M sodium acetate, flow rate: 0.6 mL/min, temperature: 35°C, standards: polyethylene glycol). The branched homopolymerized or copolymerized amino acid according to the present invention has a number-average molecular weight of about 500 g/mol-500,000 g/mol, and/or PDI in the range of about 1.0 to 4.0 (e.g., 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, and 4.0).

The salt may be an amino acid salt well-known to those skilled in the art, and preferably hydrochloride, sulfate, phosphate, carbonate, or nitrate.

In Formula I, a, b, c, d, e, and f are independently an integer of 0-6, and 1≤a+b+c+d+e+f≤20.

Preferably, a+b+c+d+e+f≤10.

Preferably, T₁, T₂, T₃, T₄, T₅, and T₆ in [] represent a random combination of the functional groups.

In a preferred embodiment of the present invention, T₁, T₂, T₃, T₄, T₅, and T₆ are independently selected from the group consisting of any one of the following structures (in the present invention, symbol " 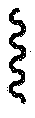 " or " 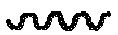 " denotes the attachment point of the shown group/ structure to the remainder of Formula I) : 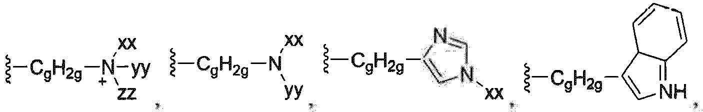 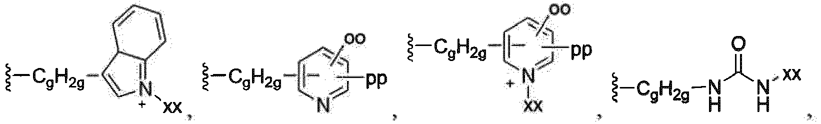 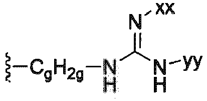 or salts thereof, 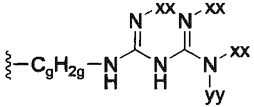 or salts thereof, 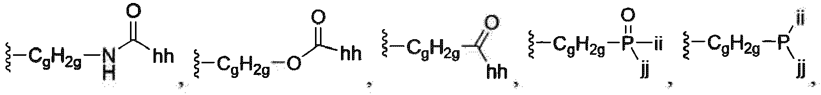 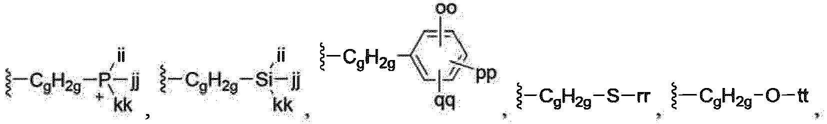 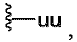 wherein, g is an integer of 0 to 10 (e.g., 0, 1, 2, 3, 4, 5, 6, 7, 8, 9, 10); wherein, xx, yy, and zz are independently selected from the group consisting of hydrogen, C1∼C18 (preferably C1-C12, more preferably C1-C8, such as C1, C2, C3, C4, C5, C6, C7, or C8) alkyl, C6∼C30 (preferably C6-C18, more preferably C6-C12) aryl, C3∼C18 (preferably C3-C12, more preferably C3-C8) cycloalkyl, carbonyl derivatives; hh is independently selected from the group consisting of hydrogen, hydroxyl, amino, halogen, C1∼C18 (preferably C1-C12, more preferably C1-C8, such as C1, C2, C3, C4, C5, C6, C7, or C8) alkyl, C6-C30 (preferably C6-C18, more preferably C6-C12) aryl, C3∼C18 (preferably C3-C12, more preferably C3-C8) cycloalkyl, amine and derivatives thereof, alkoxy derivatives, alkylthio derivatives; ii, jj, and kk are independently selected from the group consisting of hydrogen, C1∼C18 (preferably C1-C12, more preferably C1-C8, such as C1, C2, C3, C4, C5, C6, C7, or C8) alkyl, C6∼C30 (preferably C6-C18, more preferably C6-C12) aryl, C3∼C18 (preferably C3-C12, more preferably C3-C8) cycloalkyl, alkoxy and derivatives thereof; oo, pp, and qq are independently selected from the group consisting of hydrogen, carboxyl, hydroxyl, amino, C1∼C18 (preferably C1-C12, more preferably C1-C8, such as C1, C2, C3, C4, C5, C6, C7, or C8) alkyl, C6∼C30 (preferably C6-C18, more preferably C6-C12) aryl, C3∼C18 (preferably C3-C12, more preferably C3-C8) cycloalkyl, halogen, amine and derivatives thereof, alkoxy derivatives, carbonyl derivatives; rr, and tt are independently selected from the group consisting of hydrogen, C1∼C18 (preferably C1-C12, more preferably C1-C8, such as C1, C2, C3, C4, C5, C6, C7, or C8) alkyl, C6∼C30 (preferably C6-C18, more preferably C6-C12) aryl, C3∼C18 (preferably C3-C12, more preferably C3-C8) cycloalkyl, alkylthio derivatives, alkoxy derivatives, carbonyl derivatives; uu is independently selected from the group consisting of one or more of the structures represented by the following formulae: CₙH₂ₙ₊₁₋ₕTₕ (n is an integer of 0 to 10, such as 0, 1, 2, 3, 4, 5, 6, 7, 8, 9, or 10), CₙH₂ₙ₋₁₋ₕTₕ (n is an integer of 2 to 10, such as 2, 3, 4, 5, 6, 7, 8, 9, or 10), CₙH₂ₙ₋₃₋ₕTₕ (n is an integer of 2 to 10, such as 2, 3, 4, 5, 6, 7, 8, 9, or 10), CₙH₂ₙ₋₇₋ₕTₕ (n is an integer of 6 to 18, such as 6, 7, 8, 9, 10, 11, 12, 13, 14, 15, 16, 17, or 18), wherein h is an integer of 0 to 3 (such as 0, 1, 2, or 3), T is independently selected from the group consisting of any one or more of halogen (e.g., fluorine, chlorine, bromine, or iodine).

In a preferred embodiment of the present invention, T₁, T₂, T₃, T₄, T₅, and T₆ are independently selected from the group consisting of hydrogen, hydroxyl, mercapto, amino, carboxyl, C1∼C18 alkyl and derivatives thereof, C6∼C30 aryl and derivatives thereof, C3∼C8 cycloalkyl and derivatives thereof, C2∼C8 alkenyl and derivatives thereof, C2∼C8 alkynyl and derivatives thereof, C1 ∼ C8 alkoxy and derivatives thereof, C1 ∼ C8 carboxylic acid and derivatives thereof, C1 ∼ C8 amine and derivatives thereof, C2 ∼ C8 nitrogen-containing heterocyclic group and derivatives thereof, C2∼C8 oxygen-containing heterocyclic group and derivatives thereof, or C2∼C8 sulfur-containing heterocyclic group and derivatives thereof; and at least one of T₁, T₂, T₃, T₄, T₅, and T₆ is selected from the group consisting of hydroxyl, mercapto, amino, carboxyl, C2 ∼ C8 alkenyl and derivatives thereof, C2 ∼ C8 alkynyl and derivatives thereof, C1 ∼ C8 alkoxy and derivatives thereof, C1 ∼ C8 carboxylic acid and derivatives thereof, C1 ∼ C8 amine and derivatives thereof, C2 ∼ C8 nitrogen-containing heterocyclic group and derivatives thereof, C2∼C8 oxygen-containing heterocyclic group and derivatives thereof, or sulfur-containing heterocyclic group and derivatives thereof.

The above derivatives preferably have a C1∼C5 alkyl substituent, C1∼C5 alkoxy substituent, halogen, hydroxyl, mercapto, nitro, cyano, C5∼C8 aryl, C5∼C8 heteroaryl, C3∼C5 cycloalkyl, carboxyl, amino, amido substituent, or any one or more of C atom (s) is/are substituted with O or S.

Preferably, T₁, T₂, T₃, T₄, T₅, and T₆ are independently selected from the group consisting of H, C1∼C5 alkyl, or C1∼C5 substituted alkyl; wherein the substituted alkyl preferably contains hydroxyl substituent, mercapto substituent, aryl substituent, heteroaryl substituent, carboxyl substituent, heterocyclyl substituent, amido substituent, amino substituent, or C atom (s) is/are substituted by O or S.

The carbon atom numbers of the above aryl substituent, heteroaryl substituent, and heterocyclyl substituent are preferably 5∼12, more preferably 5∼8.

The carbon atom numbers of the above carboxyl substituent, amido substituent, and amino substituent are preferably 1∼8, more preferably 1∼5.

In the present invention, preferably, the terminal group of T₁, T₂, T₃, T₄, T₅, and T₆ is independently selected from the group consisting of carboxyl, hydroxyl, amino, amido, mercapto, guanidino, or N, S, or O-containing heterocyclyl. The N-containing heterocyclyl is preferably imidazolyl or benzopyrrolyl.

More preferably, T₁, T₂, T₃, T₄, T₅, and T₆ are independently selected from the group consisting of any one of the following structures: 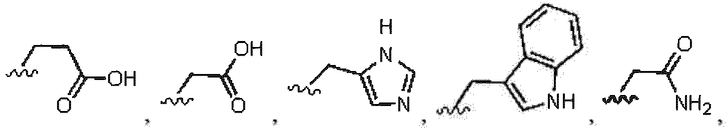 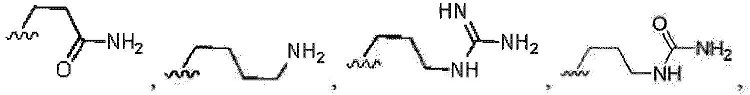 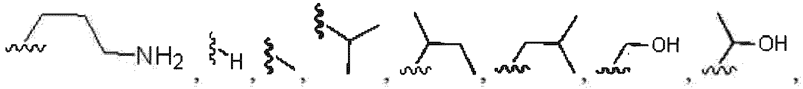 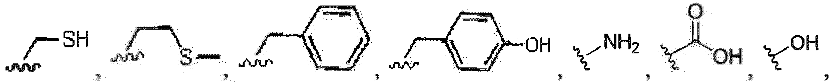 or

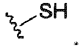

In the above poly(amino acid), when T₁, T₂, T₃, T₄, T₅, and T₆ are H at the same time, the obtained poly(amino acid) is a linear structure; when T₁, T₂, T₃, T₄, T₅, and T₆ in at least one amino acid unit are not H at the same time, the obtained poly(amino acid) is a branched structure; and when at least one amino acid unit has functionality of ≥3, a branched poly(amino acid) may be obtained.

The amino acid unit preferably includes any one or more of lysine, ornithine, arginine, glutamic acid, histidine, asparagine, glutamine, serine, tryptophan, citrulline, aspartic acid, threonine, tyrosine, cysteine, glycine, alanine, valine, leucine, isoleucine, phenylalanine, proline, and methionine.

In a preferred embodiment of the present invention, the branched poly(amino acid) is obtained by the homopolymerization of one amino acid unit, wherein the amino acid unit has a functionality of ≥3.

The amino acid unit is preferably glutamic acid, lysine, arginine, ornithine, histidine, aspartic acid, tryptophan, serine, citrulline, tyrosine, cysteine, asparagine, glutamine, or threonine. Preferably, the amino acid unit is a basic amino acid; more preferably, the amino acid unit is lysine, arginine, ornithine, or histidine.

In another preferred embodiment of the present invention, the branched poly(amino acid) is obtained by the copolymerization of two or more amino acid units, wherein the copolymerization unit at least contains one or more amino acid having functionality of ≥3; and the amino acid unit having functionality of ≥3 accounts for δ (0<δ≤100%) of the amino acid units.

In the copolymerization process, the amino acid unit having functionality of ≥3 provides the branched structure for poly(amino acid).

The copolymerized amino acid unit is preferably glutamic acid, lysine, ornithine, arginine, histidine, asparagine, glutamine, serine, tryptophan, aspartic acid, citrulline, threonine, tyrosine, or cysteine; preferably, the amino acid unit includes at least one basic amino acid unit. More preferably, the amino acid unit includes at least one or more of lysine, ornithine, arginine, and histidine.

In certain specific embodiments of the present invention, the branched poly(amino acid) is obtained by the copolymerization of arginine and alanine, or obtained by the copolymerization of ornithine and leucine, or obtained by the copolymerization of lysine and alanine, or obtained by the copolymerization of histidine and phenylalanine, or obtained by the copolymerization of lysine and arginine. More preferably, the copolymerization monomer is selected from the group consisting of the following combinations: arginine and alanine, ornithine and leucine, lysine and alanine, histidine and phenylalanine, ornithine and 6-aminohexanoic acid, lysine and arginine, phenylalanine and alanine and lysine, arginine and serine, arginine and glutamic acid, and histidine and serine.

Regarding the molar ratio of respective monomers in the copolymerized branched poly(amino acid), it is required that the amino acid unit having functionality of ≥3 accounts for greater than 0 and less than or equal to 100% (δ) in the amino acid units (i.e., 0<δ≤100%). Preferably, regarding the copolymerized amino acid obtained by the copolymerization of two amino acid units, the molar ratio of two amino acid units is in the range of 0.1:10∼10:0.1; regarding the copolymerized amino acid obtained by the copolymerization of three amino acid units, the molar ratio of three amino acid units is in the range of 0.1-10:0.1-10:0.1-10.

In the present invention, there are no particular limitations on the preparation method of the branched poly(amino acid). The branched poly(amino acid) may be prepared according to the methods well-known to those skilled in the art, preferably prepared according to the following method:
The amino acids are mixed and reacted under an atmosphere of inert gas at 25-250 °C for 1 min∼96 h, to give the branched poly(amino acid).

The inert gas is preferably nitrogen gas or argon gas.

The reaction temperature is preferably 150∼200 °C, and the reaction time is preferably 30 min∼24 h, more preferably 2 h∼12 h.

The branched structure of the poly(amino acid) results in that such material has many active functional groups, may be further modified, has a good biocompatibility, and will not develop drug resistance during the long-term use of these antimicrobial agents.

The present invention preferably includes any one or more of the following modifications to the poly(amino acid):
I. Modifying the amino group or the amino group in the amides into the following groups: 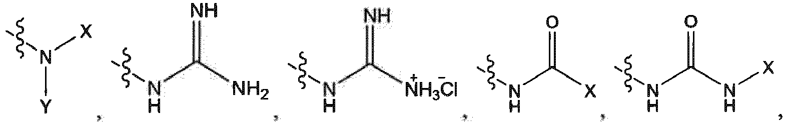 or 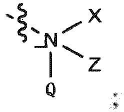
II. Modifying the hydroxyl group into -OR₁ or-OC (=O) R₂;
III. Modifying the mercapto group into -SR₃;
IV. Modifying the carboxyl group into -C (=O) NHR₄ or-C (=O) OR₅;
V. Modifying the guanidine group into the group as shown by Formula V-1;
VI. Modifying the NH in the nitrogen-containing heterocyclyl into NR₆; 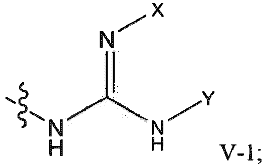
wherein, X, Y, Z, and Q are independently selected from the group consisting of hydrogen, C1∼C18 alkyl and derivatives thereof, C6∼C30 aryl and derivatives thereof, C3∼C18 cycloalkyl and derivatives thereof, C2∼C18 alkenyl and derivatives thereof, C2∼C18 alkynyl and derivatives thereof, C1∼C18 alkoxy and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof;

R₁, R₂, R₃, R₄, R₅, and R₆ are independently selected from the group consisting of H, C1∼C18 alkyl and derivatives thereof, C6∼C30 aryl and derivatives thereof, C3∼C18 cycloalkyl and derivatives thereof, C2∼C18 alkenyl and derivatives thereof, C2∼C18 alkynyl and derivatives thereof, C1∼C18 alkoxy and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof; and R₁, R₃, R₅, R₆ are not H.

The above derivatives are preferably C1∼C5 alkyl substituent, C1∼C5 alkoxy substituent, halogen, hydroxyl, mercapto, nitro, cyano, C5∼C8 aryl, C5∼C8 heteroaryl, C3∼C5 cycloalkyl, carboxyl, amino, amido substituent, or any one or more of C atom (s) is/are substituted by O or S.

More preferably, X, Y, Z, and Q are independently selected from the group consisting of hydrogen, C1∼C3 alkyl, C6∼C8 aryl, C3-C6 cycloalkyl, C1∼C3 alkoxy, C2∼C5 nitrogen-containing heterocyclic group, C2-C5 oxygen-containing heterocyclic group, or C2∼C5 sulfur-containing heterocyclic group;

R₁, R₂, R₃, R.₄, R₅, and R₆ are independently selected from the group consisting of C1∼C3 alkyl, C6∼C8 aryl, C3∼C6 cycloalkyl, C1∼C3 alkoxy, C2∼C5 nitrogen-containing heterocyclic group, C2∼C5 oxygen-containing heterocyclic group, or C2∼C5 sulfur-containing heterocyclic group.

In certain specific examples of the present invention, X, Y, Z, Q, R₁, R₂, R₃, R₄, R₅, and R₆ are independently selected from the group consisting of hydrogen, methyl, ethyl, butyl, isopropyl, acetyl, formyl, etc.

In the present invention, there are no particular limitations on the N atom number of the nitrogen-containing heterocyclyl, which may be the nitrogen-containing heterocyclic group well-known in the art, and N atom number may be but not limited to 1∼3. The modification of N atom may involve the modifications to all N atoms, or the modifications to 1 or 2 N atoms.

In certain specific examples of the present invention, the nitrogen-containing heterocyclyl is imidazolyl, which is modified to the group shown by Formula VI-1:

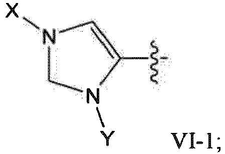

The ranges of the above X and Y are the same as recited above, and will not be repeated here.

Preferably, the poly(amino acid) of the present invention may be guanidino-modified, quaternary ammonium salt-modified, acetyl-modified, ether group-modified, methyl ester-modified, or hydroxyl-modified poly(amino acid) (homopolymerized or copolymerized amino acid), more preferably selected from the group consisting of: guanidino-modified hyperbranched polyornithine, quaternary ammonium salt-modified hyperbranched polyornithine, acetyl-modified hyperbranched polylysine, guanidino-modified ε-polylysine, guanidino-modified α-polylysine, ether group-modified poly (arginine-serine), methyl ester-modified poly (arginine-glutamic acid), hydroxyl-modified poly (ornithine-cysteine), ether group-modified poly (histidine-serine), guanidino-modified poly (ornithine and leucine), guanidino-modified poly (lysine-alanine), and quaternary ammonium salt-modified poly (lysine-alanine).

The above modifications may improve the antimicrobial performances of the poly(amino acid) and reduce the hemolysis ratio and cytotoxicity.

In the present invention, there are no particular limitations on the above modification method, as long as the applied method is well-known to those skilled in the art.

In the present invention, preferably, the antimicrobial agents may also comprise an adjuvant, and the amount of the adjuvant is preferably 0∼99wt%.

In the present invention, there are no particular limitations on the type of the adjuvant, which may be an adjuvant appropriate for the antimicrobial agents and well-known to those skilled in the art.

The adjuvant for the antimicrobial preferably includes: [i] inorganic antimicrobial agents such as metals, metal ions, and metal salts and oxides thereof; [ii] organic antimicrobial agents such as organic metals, organic halides, guanidines, organic nitro compounds, organophosphorus and organoarsenic compounds, furan and derivatives thereof, pyrroles, imidazoles, acyl anilides, thiazole and derivatives thereof, and quaternary ammonium salts; [iii] one or more of natural antimicrobial agents such as natural antimicrobial peptides and macromolecular saccharides; [iv] non-toxic additives or carriers such as glycerine, PEG, macromolecular saccharides, polypeptides, plastic, ceramic, glass, apatite, resin, fiber, and rubber.

More preferably, the adjuvant for the antimicrobial agent is one or more of metal Ti, Ag⁺, Cu²⁺, Fe³⁺, Zn²⁺, quaternary ammonium salts, halogenoamines, polybiguanides, halogenated phenols, chitosan, protamine, and natural antimicrobial peptides.

In certain specific embodiments of the present invention, the adjuvant is polyhexamethylene biguanidine.

In the present invention, there are no particular limitations on the dosage form of the antimicrobial agent, which may be used in one or more forms selected from the group consisting of solid, solution, suspension, emulsion, hydrogel, oleogel, aerosol, being coated or grafted onto the solid surface, or being blended with other materials.

In the present invention, there are no particular limitations on the preparation method of the antimicrobial agent, as long as the branched poly(amino acid) is mixed with the adjuvant.

The mixing process may or may not make use of a solvent. The solvent may be water or organic solvent.

The organic solvent is preferably one or more of methanol, ethanol, ethyl acetate, n-heptane, dimethyl formamide, dimethyl acetamide, tetrahydrofuran, chloroform, dichloromethane, tetrachloromethane, acetonitrile, petroleum ether, n-hexane, cyclohexane, dioxane, dimethyl sulfoxide, xylene, toluene, benzene, chlorobenzene, bromobenzene, acetone, and ionic liquids.

The above antimicrobial agent provided in the present invention has a simple preparation process, low equipment requirements, easy operations, easy material availability, low costs, good prospect for industrial application, and a broad-spectrum antimicrobial property on microbes.

The present invention also provides the use of the above antimicrobial agent in the antimicrobial field. There are no particular limitations on the antimicrobial range, which may be an antimicrobial range well-known to those skilled in the art.

The antimicrobial range preferably includes the uses in the inhibition of one or more of bacteria, virus, fungus, actinomyces, rickettsia, mycoplasma, chlamydia, and spirochete.

The above antimicrobial agent may be applied to various application fields well-known to those skilled in the art, without any particular limitations.

The application fields are preferably the fields of foods, cosmetics, medical products, and healthcare products.

For example, it may be used in the food preservatives, food antistaling agents, cosmetic additives, mouthwashes, disinfectants, multifunctional care solutions, preservatives in eye drops, swimming pool disinfectants, toothpastes, facial cleansers, hand sanitizers, disinfectant soaps, and disinfectants and preservatives for fruit and vegetable storage.

Compared to prior art, the present invention provides a branched poly(amino acid) antimicrobial agent, comprising a branched poly(amino acid); the branched poly(amino acid) is obtained by the homopolymerization of one amino acid unit, or obtained by the copolymerization of two or more amino acid units; and the amino acid unit has a structure shown by Formula I. The present invention utilizes amino acids as raw material, is non-toxic, has no side effects, and is a green and environmentally friendly new antimicrobial agent that is acceptable to the users. In particular, the branched structure of the poly(amino acid) makes such material have many active functional groups, may be further modified, and have a good biocompatibility, and will not develop drug resistance during the long-term use of this antimicrobial agent.

### BRIEF DESCRIPTION OF THE DRAWINGS

Figure 1 is the ¹H NMR spectrum of the branched polylysine prepared in Example 2 of the present invention;
Figure 2 is the ¹H NMR spectrum of the branched polyarginine prepared in Example 10 of the present invention; and
Figure 3 is the ¹H NMR spectrum of the branched poly(amino acid) prepared in Example 25 of the present invention.

### DETAILED DESCRIPTION OF THE INVENTION

In order to further illustrate the present invention, the branched poly(amino acid) antimicrobial agent provided by the present invention and its use will be described in detail below in conjunction with Examples. Unless otherwise specified, the reaction raw materials used in the following Examples are all commercially available products, and purchased from Shanghai Aladdin Biochemical Technology Co., Ltd., Sigma-Aldrich Chemical Reagent Co., Ltd., J&K Bailingwei Technology Co., Ltd., Shanghai McLean Biochemical Technology Co., Ltd. or Sinopharm Group Chemical Reagent Co., Ltd.

In the following Examples, the molecular weight of the polymer is measured by gel-permeation chromatograph (GPC), and the specific measurement method is as follows: the molecular weight (*M*ₙ) of the polymer and the distribution thereof (PDI=*M*_{w}/*M*ₙ) are measured by Waters 2414 gel-permeation chromatograph system equipped with Waters 2414 interference refraction detector (mobile phase: 0.2M acetic acid/0.1M sodium acetate, flow rate: 0.6 mL/min, temperature: 35°C, standards: polyethylene glycol).

### Example 1

100 grams of arginine was added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in diethyl ether, to give 82.7 grams of hyperbranched polyarginine as light yellow solid powder. GPC characterization: *M*ₙ= 2200 g/mol, PDI=1.91.

### Example 2

91.32 grams of lysine hydrochloride and 28.05 grams of KOH were added to a 500 mL round bottom flask, and a water separator was connected. The nitrogen purge was conducted for three times (each for more than 10 minutes) so as to finally maintain nitrogen atmosphere. The reaction was conducted at 250 °C under stirring and heating for 1 minute. After the stop of heating, the polymer was dissolved with methanol and filtered to remove salts, concentrated, then precipitated in diethyl ether, to give 84 grams of hyperbranched polylysine as light yellow solid powder. GPC characterization: *M*ₙ=1100 g/mol, PDI=1.81.

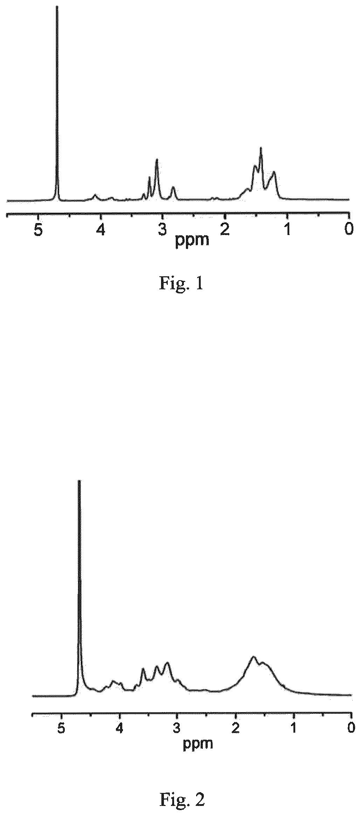

Figure 1 shows the ¹H NMR spectrum of the synthesized branched poly(amino acid).

### Example 3

50 grams of serine and 50 mL of n-hexanol were added to a 500 mL round bottom flask, and a water separator was connected. Under nitrogen atmosphere, the reaction was conducted at 190 °C under stirring and heating for 10 hours. After the stop of heating, the reaction system was cooled to room temperature. The polymer was dissolved with ethanol and precipitated in diethyl ether, to give 28.5 grams of hyperbranched polyserine as light yellow solid powder. GPC characterization: *M*ₙ=7800 g/mol, PDI=1.71.

### Example 4

91.32 grams of lysine hydrochloride, 28.05 grams of KOH, and 10 mg of antimony trioxide were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 96 hours. After the stop of heating, the reaction system was cooled to room temperature. The polymer was dissolved with methanol and filtered to remove salts, concentrated, then precipitated in diethyl ether, to give 55.5 grams of hyperbranched polylysine as light yellow solid powder. GPC characterization: *M*ₙ= 500000 g/mol, PDI=2.36.

### Example 5

80 grams of lysine, 20 grams of lysine hydrochloride, and 6.14g KOH were added to a 500 mL round bottom flask, and a water separator was connected. The nitrogen purge was conducted for three times (each for more than 10 minutes) so as to finally maintain nitrogen atmosphere. The reaction was conducted at 100 °C under stirring and heating for 10 hours. After the stop of heating, the reaction system was cooled to room temperature. The polymer was dissolved with ethanol and filtered to remove salts, concentrated, then precipitated in diethyl ether, to give 68.5 grams of hyperbranched polylysine as brown solid powder. GPC characterization: *M*ₙ= 800 g/mol, PDI=1.66.

### Example 6

50 grams of cysteine and 100 mL of DMF were added to a 500 mL round bottom flask, and a water separator was connected. Under nitrogen atmosphere, the reaction was conducted at 180 °C under stirring and heating for 10 hours. After the stop of heating, the reaction system was cooled to room temperature. The polymer was dissolved with methanol and precipitated in diethyl ether, to give 33.5 grams of hyperbranched polycysteine as yellow solid powder. GPC characterization: *M*ₙ=1900 g/mol, PDI=2.07.

### Example 7

50 grams of glutamic acid and 100 mL of ethylene glycol were added to a 500 mL round bottom flask, and a water separator was connected. Under nitrogen atmosphere, the reaction was conducted at 200 °C under stirring and heating for 1 minute. After the stop of heating, the reaction system was cooled to room temperature. The polymer was dissolved with methanol and precipitated in diethyl ether, to give 31.5 grams of hyperbranched polyglutamic acid as pale yellow solid powder. GPC characterization: *M*ₙ=2100 g/mol, PDI=1.86.

### Example 8

50 grams of arginine and 100 mL of ethylene glycol were added to a 500 mL round bottom flask, and a water separator was connected. Under nitrogen atmosphere, the reaction was conducted at 150 °C under stirring and heating for 96 hours. After the stop of heating, the reaction system was cooled to room temperature. The polymer was dissolved with methanol and precipitated in ethyl acetate, to give 34.5 grams of hyperbranched polyarginine as light yellow solid powder. GPC characterization: *M*ₙ= 1800 g/mol, PDI=2.18.

### Example 9

100 grams of lysine and 0.1 grams of phosphoric acid were added to a 500 mL round bottom flask, and a water separator was connected. The nitrogen purge was conducted for three times (each for more than 10 minutes) so as to finally maintain nitrogen atmosphere. The reaction was conducted at 100 °C under stirring and heating for 96 hours. After the stop of heating, the reaction system was cooled to room temperature. The polymer was dissolved with methanol and precipitated in diethyl ether, to give 78.5 grams of hyperbranched polylysine as tawny solid powder. GPC characterization: *M*ₙ= 6800 g/mol, PDI=1.97.

### Example 10

100 grams of arginine and 200 grams of ethylene glycol were added to a 500 mL round bottom flask, and a water separator was connected. N₂ was bubbled for 30 min. The nitrogen purge was conducted for three times (each for more than 10 minutes) so as to finally maintain nitrogen atmosphere. The reaction was conducted at 170 °C under stirring and heating for 8 hours. After the stop of heating, the reaction system was cooled to room temperature. The ethylene glycol was separated, and the polymer was precipitated in diethyl ether, to give 71.2 grams of hyperbranched polyarginine as light yellow solid powder. GPC characterization: *M*ₙ= 3100 g/mol, PDI=1.78.

Figure 2 shows the ¹H NMR spectrum of the synthesized branched poly(amino acid).

### Example 11

100 grams of histidine and 200 grams of ethylene glycol were added to a 500 mL round bottom flask, and a water separator was connected. N₂ was bubbled for 30 min. The nitrogen purge was conducted for three times (each for more than 10 minutes) so as to finally maintain nitrogen atmosphere. The reaction was conducted at 180°C under stirring and heating for 24 hours. After the stop of heating, the reaction system was cooled to room temperature. The ethylene glycol was separated, and the polymer was washed with diethyl ether for 5 times, to give 71.2 grams of hyperbranched polyhistidine as yellow solid powder. GPC characterization: *M*ₙ=1500 g/mol, PDI=1.71.

### Example 12

100 grams of ornithine and 50 g of water were added to a 500 mL round bottom flask, and a water separator was connected. The nitrogen purge was conducted for three times (each for more than 10 minutes) so as to finally maintain nitrogen atmosphere. The reaction was conducted at 150 °C under stirring and heating for 5 hours. After the stop of heating, the polymer was grounded to give 87 grams of hyperbranched polylysine as brown solid powder. GPC characterization: *M*ₙ= 3400 g/mol, PDI=1.77.

### Example 13

2 g of hyperbranched polyarginine of Example 10 and 0.2 g of silver nitrate were dissolved into 5 mL of water, stirred and uniformly dispersed, then freeze-dried, to give 2.18 g of a mixture of hyperbranched polyarginine with silver ions.

### Example 14

100 grams of lysine and 50 g of water were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 5 hours. After the stop of heating, the polymer was grounded to give 87 grams of hyperbranched polylysine as brown solid powder. GPC characterization: *M*ₙ= 2400 g/mol, PDI=1.77.

### Example 15

2 g of hyperbranched polylysine of Example 14 and 0.2 g of chitosan were dissolved into 5 mL of water, stirred and uniformly dispersed, then freeze-dried, to give 2.18 g of a mixture of hyperbranched polylysine and chitosan.

### Example 16

100 grams of citrulline and 50 g of water were added to a 500 mL round bottom flask, and a water separator was connected. The nitrogen purge was conducted for three times (each for more than 10 minutes) so as to finally maintain nitrogen atmosphere. The reaction was conducted at 190 °C under stirring and heating for 5 hours. After the stop of heating, the polymer was dissolved with water and dialyzed by secondary water, to give 57 grams of hyperbranched polycitrulline as light yellow solid powder. GPC characterization: *M*ₙ= 5700 g/mol, PDI=1.27.

### Example 17

100 grams of (2S,3R,4S)-α-(carboxylcyclopropyl) glycine and 50 g of water were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 5 hours. After the stop of heating, the polymer was grounded to give 86.4 grams of hyperbranched poly(amino acid) as brown solid powder. GPC characterization: *M*ₙ= 7500 g/mol, PDI=1.87.

### Example 18

100 grams of 5-amino-2-hydrazinopentanoic acid (CAS: 60733-16-6) and 50 g of water were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 5 hours. After the stop of heating, the polymer was grounded to give 83.4 grams of hyperbranched poly(amino acid) as brown solid powder. GPC characterization: *M*ₙ= 8400 g/mol, PDI=1.94.

### Example 19

100 grams of 4-amino-3-hydroxylbutanoic acid and 50 g of water were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 5 hours. After the stop of heating, the polymer was fractionated by gel column to give 78.4 grams of hyperbranched poly(amino acid) as a brown solid powder. GPC characterization: *M*ₙ= 7300 g/mol, PDI=1.48.

### Example 20

36 mg of hyperbranched poly(amino acid)s prepared in Examples 1-19 were respectively weighted and dissolved into 3 mL of sterile PBS, to give 12 mg/mL of stock solution. The antimicrobial activity of the hyperbranched poly(amino acid)-based antimicrobial agents was measured in accordance with the following method, and a part of experiment results were shown in Table 1.

The various strains used in the following Examples were purchased from the National Institute for the Control of Biological Products.

The antimicrobial activity of the hyperbranched poly(amino acid)-based antimicrobial agents was tested through the 96-well plate method, and *ε*-polylysine synthesized by fermentation was used as the control to evaluate the antibacterial capacity of the resulting hyperbranched poly(amino acid)-based antimicrobial agents. The minimum inhibitory concentration (MIC) is defined as the lowest polymer concentration that inhibits microbial growth by 90% compared to the control group.

A small amount of strains were picked from the agar slant medium with the inoculating ring to the ordinary MH medium, incubated at 37°C overnight to recover the strains and achieve exponential growth. The microbial solution was diluted so that the concentration of the microbial solution was 10⁶ CFU/mL. In each well, 175 µL of microbial solution and 25 µL of polymer solutions at different concentrations were added. The 96-well plate was incubated at 37°C for 20 hours, and the OD₆₀₀ value was measured by the microplate reader.

**Table 1. Comparisons of MIC values of different antimicrobial polymers against different microbes**

| Strain Name | | MIC values of different antimicrobial polymers against different microbes (µg/mL) | | | | | | | | | | | |
|---|---|---|---|---|---|---|---|---|---|---|---|---|---|
| | | Ex. 1 | Ex. 5 | Ex. 9 | Ex. 10 | Ex. 11 | Ex. 12 | Ex. 13 | Ex. 16 | Ex. 17 | Ex. 18 | Ex. 19 | ε-polylysin e |
| Gram-negative bacteria | *Escherichia coli* ATCC8739 | 24 | 47 | 24 | 24 | 47 | 24 | 24 | 12 | 24 | 12 | 12 | 47 |
| | *Pseudomonas aeruginosa* A TCC9207 | 24 | 24 | 12 | 12 | 47 | 24 | 12 | 12 | 12 | 12 | 24 | 47 |
| | *Klebsiella pneumoniae* A TCC700603 | 24 | 47 | 47 | 24 | 24 | 24 | 12 | 12 | 24 | 24 | 24 | 24 |
| | *Salmonella paratyphi B* CMCC50094 | 24 | 24 | 24 | 24 | 24 | 12 | 12 | 24 | 47 | 6 | 12 | 47 |
| | *Acinetobacter baumannii* ATCC 19606 | 24 | 24 | 24 | 24 | 24 | 24 | 12 | 12 | 12 | 6 | 6 | 24 |
| Gram-positive bacteria | *Staphylococcus aureus* ATCC 25923 | 12 | 12 | 12 | 12 | 24 | 12 | 24 | 12 | 6 | 24 | 12 | 24 |
| | Methicillin-resistant *Staphylococcus aureus* ATCC 43300 | 24 | 24 | 24 | 24 | 47 | 24 | 47 | 47 | 24 | 24 | 24 | 47 |
| | *Bacillus subtilis* ATCC 6633 | 12 | 12 | 12 | 12 | 24 | 12 | 24 | 6 | 47 | 12 | 12 | 24 |
| | *Micrococcus luteus* ATCC 10240 | 12 | 12 | 12 | 6 | 24 | 12 | 24 | 12 | 24 | 12 | 12 | 24 |
| | *Bacillus pumilus* ATCC700814 | 12 | 12 | 12 | 12 | 47 | 6 | 12 | 12 | 24 | 12 | 24 | 24 |
| Fungi | *Candida albicans* ATCC 10231 | 24 | 24 | 24 | 12 | 96 | 24 | 12 | 12 | 12 | 6 | 12 | 47 |
| | *Saccharomyces cerevisiae* ATCC 9763 | 24 | 47 | 47 | 24 | 47 | 24 | 12 | 12 | 47 | 12 | 24 | 47 |

### Example 21

36 mg of hyperbranched poly(amino acid)s prepared in Examples 1-19 were weighted respectively and dissolved into 3 mL of sterile PBS, to give 12 mg/mL of stock solution. The *in vitro* hemolytic activity of the hyperbranched poly(amino acid)-based antimicrobial agents was measured in accordance with the following method, and a part of experiment results were shown in Table 2.

The *in vitro* hemolytic activity of the hyperbranched poly(amino acid)-based antimicrobial agents was tested by a 96-well plate method, and *ε*-polylysine synthesized by fermentation was used as the control to evaluate the *in vitro* hemolytic activity of the resulting hyperbranched poly(amino acid)-based antimicrobial agents.

Preparation of 2% (v/v) erythrocyte suspension: 2 mL of fresh healthy human blood was taken and dilute with 10 mL of endotoxin-free PBS buffer solution. The blood was transferred to a triangular flask with glass beads and shaken for 10 minutes, or stirred with a glass rod, to remove the fibrin and make it into deflbrinated blood. The deflbrinated blood was centrifuged at 1000∼1500 r/min at 20 °C for 10∼15 minutes, and the supernatant was removed. The precipitated erythrocyte was then washed with the PBS buffer solution for 4 times in accordance with the above method, until the supernatant does not appear red. The obtained erythrocyte was formulated into a 2% suspension with physiological saline for subsequent experiments.

The polymer was diluted with the PBS buffer solution to prepare solutions of different concentrations and added to 96-well plates. The PBS buffer solution alone was used as the negative control, and 0.2% Triton-X-100 dissolved in water was used as the positive control for *in vitro* hemolytic activity test. The washed erythrocyte (2% v/v, 50 µL) was added to the 96-well plate, mixed thoroughly and incubated. The absorption at 540 nm was measured with a microplate reader.

**Table 2. Test results for the in vitro hemolytic activities of different antimicrobial polymers**

| Concentration of the antimicrobial polymer (µg/mL) | Hemolysis ratio (%) | | | | | | | | | | | |
|---|---|---|---|---|---|---|---|---|---|---|---|---|
| | Ex. 1 | Ex. 5 | Ex. 9 | Ex. 10 | Ex. 11 | Ex. 12 | Ex. 13 | Ex. 16 | Ex. 17 | Ex. 18 | Ex. 19 | *ε-*polylysine |
| 3 | 0 | 0 | 0 | 0 | 0 | 0 | 0 | 0 | 0 | 0 | 0 | 0 |
| 6 | 0 | 0 | 0 | 0 | 0 | 0 | 0 | 0 | 0 | 0 | 0 | 0 |
| 12 | 0 | 0 | 0 | 0 | 0 | 0 | 0 | 0 | 0 | 0 | 0 | 0 |
| 24 | 0 | 0 | 0 | 0 | 0 | 0 | 0 | 0 | 0 | 0 | 0 | 0 |
| 47 | 0 | 0 | 0 | 0 | 0 | 0 | 0 | 5 | 0 | 0 | 0 | 0 |
| 94 | 0 | 0 | 0 | 0 | 1 | 0 | 0 | 8 | 0 | 0 | 0 | 0 |
| 188 | 0 | 0 | 0 | 0 | 3 | 0 | 0 | 12 | 0 | 0 | 0 | 0 |
| 375 | 0 | 1 | 0 | 0 | 10 | 0 | 0 | 19 | 0 | 0 | 0 | 0 |
| 750 | 0 | 2 | 1 | 0 | 25 | 0 | 1 | 32 | 0 | 0 | 0 | 0 |
| 1500 | 1 | 3 | 2 | 1 | 45 | 0 | 1 | 54 | 1 | 0 | 0 | 0 |

### Example 22

The Example was used to test the *in vivo* acute toxicity of the hyperbranched poly(amino acid)-based antimicrobial agents in animals, and *ε*-polylysine synthesized by fermentation was used as the control to evaluate the *in vivo* acute toxicity of the resulting hyperbranched poly(amino acid)-based antimicrobial agents in animals.

One hundred mice (Balb/C mice, purchased from Jilin University), half male and half female, weighing 21±3 g, were used. The hyperbranched poly(amino acid)-based antimicrobial agents prepared in Examples 1-19 were taken at a dose of 1 mg/mL respectively and intramuscularly injected into mice once a day for 15 consecutive days to observe the toxic reaction of mice. The experimental results showed that after intramuscular injection for 21 consecutive days, except for few mice with reduced vitality, the rest had no significant abnormal reactions, and all mice survived. It was demonstrated that the resulting hyperbranched poly(amino acid)-based antimicrobial agents have lower toxicity *in vivo.*

### Example 23

80 grams of arginine and 20 grams of alanine were added to a 500 mL round bottom flask, and a water separator was connected. The nitrogen purge was conducted for three times (each for more than 10 minutes) so as to finally maintain nitrogen atmosphere. The reaction was conducted under stirring and heating at 160 °C for 5 hours. After the stop of heating, the reaction system was cooled to room temperature. The polymer was dissolved with ethanol and precipitated in diethyl ether, to give 78.7 grams of hyperbranched poly(amino acid) as light yellow solid powder. GPC characterization: *M*ₙ=3100 g/mol, PDI=1.76.

### Example 24

50 grams of ornithine, 50 grams of leucine, and 10 mg of scandium trifluoromethylsulfonate were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 2 hours. After the stop of heating, the polymer was dissolved with methanol and precipitated in diethyl ether, to give 81.5 grams of hyperbranched poly(amino acid) as dark yellow solid powder. GPC characterization: *M*ₙ=500 g/mol, PDI=1.82.

### Example 25

91.32 grams of lysine hydrochloride, 28.05 grams of KOH, and 20 grams of alanine were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 10 hours. After the stop of heating, the reaction system was cooled to room temperature. The polymer was dissolved with methanol and precipitated in diethyl ether, to give 75.2 grams of branched poly(amino acid) as light yellow solid powder. GPC characterization: *M*ₙ=14100 g/mol, PDI=2.72.

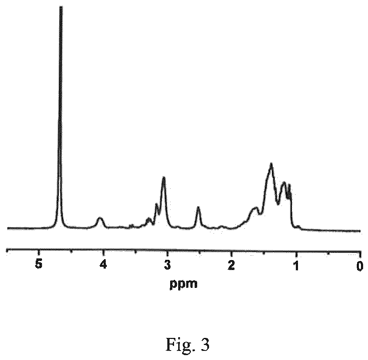

Figure 3 shows the ¹H NMR spectrum of the synthesized branched poly(amino acid).

### Example 26

90 grams of histidine, 10 grams of phenylalanine, and 10 mg of ferric trichloride were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 2 hours. After the stop of heating, the reaction system was cooled to room temperature. The polymer was dissolved with water and precipitated in tetrahydrofuran, to give 79.5 grams of hyperbranched poly(amino acid) as light yellow solid powder. GPC characterization: *M*ₙ=1100 g/mol, PDI=1.62.

### Example 27

80 grams of ornithine was first added to a 500 mL round bottom flask, and a water separator was connected. The nitrogen purge was conducted for three times (each for more than 10 minutes) so as to finally maintain nitrogen atmosphere. The reaction was conducted at 190 °C under stirring and heating for 4 hours. Then 20 grains of 6-aminohexanoic acid was added to the reaction system and reacted for another 4 hours. After the stop of heating, the reaction system was cooled to room temperature. The polymer was dissolved with methanol and precipitated in diethyl ether, to give 82.3 grams of hyperbranched poly(amino acid) having a core-shell structure, as light yellow solid powder. GPC characterization: *M*ₙ=11100 g/mol, PDI=1.94.

### Example 28

91.32 grams of lysine hydrochloride, 20 grams of NaOH, and 20 grams of alanine were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 36 hours. After the stop of heating, the reaction system was cooled to room temperature. The polymer was dissolved with methanol and precipitated in diethyl ether, to give 74.8 grams of hyperbranched poly(amino acid) as light yellow solid powder. GPC characterization: *M*ₙ=81100 g/mol, PDI=3.98.

### Example 29

91.32 grams of lysine hydrochloride, 20 grams of NaOH, and 20 grams of alanine were added to a 500 mL round bottom flask, and a water separator was connected. The nitrogen purge was conducted for three times (each for more than 10 minutes) so as to finally maintain nitrogen atmosphere. The reaction was conducted at 100 °C under stirring and heating for 96 hours. After the stop of heating, the reaction system was cooled to room temperature. The polymer was dissolved with methanol and precipitated in diethyl ether, to give 72.8 grams of hyperbranched poly(amino acid) as dark yellow solid powder. GPC characterization: *M*ₙ=481100 g/mol, PDI=2.21.

### Example 30

91.32 grams of lysine hydrochloride and 20 grams of NaOH were first added to a 500 mL round bottom flask, and a water separator was connected. The nitrogen purge was conducted for three times (each for more than 10 minutes) so as to finally maintain nitrogen atmosphere. The reaction was conducted at 250 °C under stirring and beating for 0.5 h. Then 20 grams of alanine was added to the reaction system and reacted for another 0.5 h. After the stop of heating, the reaction system was cooled to room temperature. The polymer was dissolved with methanol and precipitated in diethyl ether, to give 73.1 grams of hyperbranched poly(amino acid) having a core-shell structure, as light yellow solid powder. GPC characterization: *M*ₙ=1200 g/mol, PDI=1.51.

### Example 31

50 grams of lysine and 50 grams of arginine were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in ethyl acetate, to give 80.1 grams of branched poly(amino acid) as light yellow solid powder. GPC characterization: *M*ₙ=3200 g/mol, PDI=1.73.

### Example 32

80 grams of (2S,3R,4S)-α-(carboxylcyclopropyl)glycine and 20 grams of alanine were added to a 500 mL round bottom flask and 80 g of secondary water was added for dissolution, and a water separator was connected. The nitrogen purge was conducted for three times (each for more than 10 minutes) so as to finally maintain nitrogen atmosphere. The reaction was conducted at 180 °C under stirring and heating for 5 hours. After the stop of heating, the polymer was ground, to give 86.4 grams of hyperbranched poly(amino acid) as brown solid powder. GPC characterization: *M*ₙ=5000g/mol, PDI=1.88.

### Example 33

80 grams of 5-amino-2-hydrazinopentanoic acid (CAS:60733-16-6) and 20 grams of alanine were added to a 500 mL round bottom flask and 80 g of secondary water was added for dissolution, and a water separator was connected. The nitrogen purge was conducted for three times (each for more than 10 minutes) so as to finally maintain nitrogen atmosphere. The reaction was conducted at 160 °C under stirring and heating for 10 hours. After the stop of heating, the polymer was ground, to give 84.5 grams of hyperbranched poly(amino acid) as brown solid powder. GPC characterization: *M*ₙ=8700g/mol, PDI=1.92.

### Example 34

20 grams of phenylalanine and 10 grams of alanine were first added to a 500 mL round bottom flask, and a water separator was connected. The nitrogen purge was conducted for three times (each for more than 10 minutes) so as to finally maintain nitrogen atmosphere. The reaction was conducted at 100 °C under stirring and heating for 30 hours. Then 70 grams of lysine hydrochloride and 20 g of KOH were added to the reaction system and reacted for another 70 hours. After the stop of heating, the reaction system was cooled to room temperature. The polymer was dissolved with methanol and precipitated in diethyl ether, to give 68.5 grams of hyperbranched poly(amino acid) having a core-shell structure, as light yellow solid powder. GPC characterization: *M*ₙ=6200 g/mol, PDI=1.88.

### Example 35

80 grams of 4-amino-3-hydroxylbutanoic acid and 20 grams of glycine were added to a 500 mL round bottom flask and 80 g of secondary water was added for dissolution, and a water separator was connected. The nitrogen purge was conducted for three times (each for more than 10 minutes) so as to finally maintain nitrogen atmosphere. The reaction was conducted at 170 °C under stirring and heating for 12 hours. After the stop of heating, the polymer was dissolved in water and precipitated in tetrahydrofuran, to give 84.5 grams of hyperbranched poly(amino acid) as brown solid powder. GPC characterization: *M*ₙ=13700g/mol, PDI=1.72.

### Example 36

2 g of hyperbranched poly(amino acid) of Example 28 and 2 g of polyhexamethylene biguanidine were dissolved into 5 mL of water, stirred and uniformly dispersed, then freeze-dried, to give 4 g of a mixture of hyperbranched poly(amino acid) with polyhexamethylene biguanidine.

### Example 37

2 g of hyperbranched poly(amino acid) of Example 24 and 2 g of polyhexamethylene biguanidine were dissolved into 5 mL of water, stirred and uniformly dispersed, then freeze-dried, to give 4 g of a mixture of hyperbranched poly(amino acid) with polyhexamethylene biguanidine.

### Example 38

36 mg of hyperbranched poly(amino acid)s prepared in Examples 23-37 were weighted respectively and dissolved into 3 mL of sterile PBS, to give 12 mg/mL of stock solution. The antimicrobial activity of the hyperbranched poly(amino acid)-based antimicrobial agents was measured in accordance with the following method, and the experiment results were shown in Tables 3-1 and 3-2. The various strains used in the following examples were purchased from the National Institute for the Control of Biological Products.

The antimicrobial activity of the hyperbranched poly(amino acid)-based antimicrobial agents was tested by a 96-well plate method, and *ε*-polylysine synthesized by fermentation was used as the control to evaluate the antimicrobial capacity of the resulting hyperbranched poly(amino acid)-based antimicrobial agents. The minimum inhibitory concentration (MIC) is defined as the lowest polymer concentration that inhibits microbial growth by 90% compared to the control group.

A small amount of strains were picked from the agar slant medium with the inoculating ring to the ordinary M-H medium, incubated at 37°C overnight to recover the strains and achieve exponential growth. The microbial solution was diluted so that the concentration of the microbial solution was 10⁶ CFU/mL. In each well, 175 µL of microbial solution and 25 µL of polymer solutions at different concentrations were added. The 96-well plate was incubated at 37°C for 20 hours, and the OD₆₀₀ value was measured by the microplate reader.

**Table 3-1. Comparisons of MIC values of different antimicrobial polymers against different microbes**

| Strain Name | | MIC values of different antimicrobial polymers against different microbes (µg/mL) | | | | | | | | | |
|---|---|---|---|---|---|---|---|---|---|---|---|
| | | Ex. 23 | Ex. 24 | Ex. 25 | Ex. 26 | Ex. 27 | Ex. 28 | Ex. 29 | Ex. 30 | Ex. 31 | *ε-*polylysine |
| | *Escherichia coli* ATCC8739 | 24 | 24 | 24 | 24 | 47 | 47 | 47 | 24 | 24 | 47 |
| Gram-negative bacteria | *Pseudomonas aeruginosa* A TCC9207 | 24 | 12 | 12 | 12 | 24 | 24 | 47 | 12 | 24 | 47 |
| | *Klebsiella pneumoniae* ATCC700603 | 24 | 24 | 24 | 24 | 24 | 47 | 96 | 24 | 24 | 24 |
| | *Salmonella paratyphi B* CMCC50094 | 24 | 24 | 24 | 24 | 24 | 24 | 47 | 47 | 24 | 47 |
| | *Acinetobacter baumannii* ATCC 19606 | 24 | 24 | 24 | 24 | 47 | 24 | 47 | 24 | 24 | 24 |
| Gram-positive bacteria | *Staphylococcus aureus* ATCC 25923 | 6 | 12 | 12 | 12 | 24 | 12 | 47 | 12 | 12 | 24 |
| | Methicillin-resistant *Staphylococcus aureus* ATCC 43300 | 12 | 24 | 24 | 24 | 47 | 24 | 96 | 24 | 24 | 47 |
| | *Bacillus subtilis* ATCC 6633 | 6 | 12 | 12 | 12 | 47 | 12 | 47 | 12 | 6 | 24 |
| | *Micrococcus luteus* ATCC 10240 | 12 | 6 | 6 | 6 | 24 | 12 | 47 | 6 | 12 | 24 |
| | *Bacillus pumilus* ATCC700814 | 6 | 12 | 12 | 12 | 24 | 12 | 47 | 12 | 6 | 24 |
| Fungi | *Candida albicans* ATCC 10231 | 24 | 12 | 12 | 12 | 47 | 24 | 96 | 12 | 24 | 47 |
| | *Saccharomyces cerevisiae* ATCC 9763 | 24 | 24 | 24 | 24 | 47 | 47 | 96 | 24 | 24 | 47 |

**Table 3-2. Comparisons of MIC values of different antimicrobial polymers against different microbes**

| Strain Name | | MIC values of different antimicrobial polymers against different microbes (µg/mL) | | | | | | |
|---|---|---|---|---|---|---|---|---|
| | | Ex. 32 | Ex. 33 | Ex. 34 | Ex. 35 | Ex. 36 | Ex. 37 | *ε-*polylysine |
| Gram-negative bacteria | *Escherichia coli* A TCC8739 | 24 | 24 | 24 | 24 | 24 | 24 | 47 |
| | *Pseudomonas aeruginosa* A TCC9207 | 12 | 12 | 24 | 12 | 12 | 24 | 47 |
| | *Klebsiella pneumoniae* A TCC700603 | 24 | 12 | 24 | 12 | 12 | 12 | 24 |
| | *Salmonella paratyphi B* CMCC50094 | 47 | 12 | 47 | 24 | 12 | 24 | 47 |
| | *Acinetobacter baumannii* ATCC 19606 | 24 | 12 | 24 | 12 | 12 | 24 | 24 |
| Gram-positive bacteria | *Staphylococcus aureus* ATCC 25923 | 12 | 6 | 12 | 6 | 6 | 12 | 24 |
| | Methicillin-resistant *Staphylococcus aureus* ATCC 43300 | 24 | 24 | 47 | 12 | 12 | 24 | 47 |
| | *Bacillus subtilis* ATCC 6633 | 12 | 24 | 24 | 12 | 12 | 12 | 24 |
| | *Micrococcus luteus* ATCC 10240 | 6 | 24 | 24 | 24 | 12 | 12 | 24 |
| | *Bacillus pumilus* A TCC700814 | 12 | 12 | 24 | 12 | 12 | 24 | 24 |
| Fungi | *Candida albicans* | 12 | 12 | 47 | 24 | 24 | 24 | 47 |
| | ATCC10231 | | | | | | | |
| | *Saccharomyces cerevisiae* ATCC 9763 | 24 | 12 | 47 | 24 | 24 | 24 | 47 |

### Example 39

36 mg of hyperbranched poly(amino acid)s prepared in Examples 23-37 were weighted respectively and dissolved into 3 mL of sterile PBS, to give 12 mg/mL of stock solution. The *in vitro* antihemolytic activity of the hyperbranched poly(amino acid)-based antimicrobial agents was measured in accordance with the following method, and the experiment results were shown in Tables 4-1 and 4-2.

The *in vitro* hemolytic activity of the hyperbranched poly(amino acid)-based antimicrobial agents was tested through the 96-well plate method, and *ε*-polylysine synthesized by fermentation was used as the control to evaluate the *in vitro* hemolytic activity of the resulting hyperbranched poly(amino acid)-based antimicrobial agents.

Preparation of 2% (v/v) erythrocyte suspension: 2 mL of fresh healthy human blood was taken and dilute with 10 mL of endotoxin-free PBS buffer solution. The blood was transferred to a triangular flask with glass beads and shaken for 10 minutes, or stirred with a glass rod, to remove the fibrin and make it into deflbrinated blood. The deflbrinated blood was centrifuged at 20 °C at 1000∼1500 r/min for 10∼15 minutes, and the supernatant was removed. The precipitated erythrocyte was then washed with the PBS buffer solution for 4 times in accordance with the above method, until the supernatant does not appear red. The obtained erythrocyte was formulated into a 2% suspension with physiological saline for subsequent experiments.

The polymer was diluted with the PBS buffer solution to prepare solutions of different concentrations and added to 96-well plates. The PBS buffer solution alone was used as the negative control, and 0.2% Triton-X-100 dissolved in water was used as the positive control for *in vitro* hemolytic activity test. The washed erythrocyte (2% v/v, 50 µL) was added to the 96-well plate, mixed thoroughly and incubated. The absorption at 540 nm was measured with a microplate reader.

**Table 4-1. Test results for the in vitro hemolytic activities of different antimicrobial polymers**

| Concentration of the antimicrobial polymer (µg/mL) | Hemolysis ratio (%) | | | | | | | |
|---|---|---|---|---|---|---|---|---|
| | Ex. 23 | Ex. 24 | Ex. 25 | Ex. 26 | Ex. 27 | Ex. 28 | Ex. 29 | *ε*-polylysine |
| 3 | 0 | 0 | 0 | 0 | 0 | 0 | 0 | 0 |
| 6 | 0 | 0 | 0 | 0 | 0 | 0 | 0 | 0 |
| 12 | 0 | 0 | 0 | 0 | 0 | 0 | 0 | 0 |
| 24 | 0 | 0 | 0 | 0 | 0 | 0 | 0 | 0 |
| 47 | 0 | 1 | 0 | 1 | 1 | 0 | 1 | 0 |
| 94 | 1 | 4 | 0 | 4 | 4 | 0 | 4 | 0 |
| 188 | 3 | 7 | 0 | 7 | 7 | 0 | 7 | 0 |
| 375 | 6 | 12 | 2 | 12 | 12 | 0 | 12 | 0 |
| 750 | 8 | 20 | 6 | 20 | 20 | 1 | 20 | 0 |
| 1500 | 15 | 24 | 9 | 24 | 24 | 3 | 24 | 0 |

**Table 4-2. Test results for the in vitro hemolytic activities of different antimicrobial polymers**

| Concentrati on of the antimicrobial polymer (µg/mL) | Hemolysis ratio (%) | | | | | | | | |
|---|---|---|---|---|---|---|---|---|---|
| | Ex. 30 | Ex. 31 | Ex. 32 | Ex. 33 | Ex. 34 | Ex. 35 | Ex. 36 | Ex. 37 | *ε-*polylysine |
| 3 | 0 | 0 | 0 | 0 | 0 | 0 | 0 | 0 | 0 |
| 6 | 0 | 0 | 0 | 0 | 0 | 0 | 0 | 0 | 0 |
| 12 | 0 | 0 | 0 | 0 | 0 | 0 | 0 | 0 | 0 |
| 24 | 0 | 0 | 0 | 0 | 0 | 0 | 0 | 0 | 0 |
| 47 | 0 | 0 | 0 | 0 | 2 | 0 | 0 | 0 | 0 |
| 94 | 0 | 0 | 0 | 1 | 3 | 0 | 0 | 0 | 0 |
| 188 | 2 | 1 | 1 | 3 | 8 | 0 | 0 | 0 | 0 |
| 375 | 5 | 3 | 6 | 11 | 11 | 0 | 0 | 0 | 0 |
| 750 | 8 | 5 | 9 | 14 | 12 | 1 | 1 | 1 | 0 |
| 1500 | 13 | 10 | 12 | 20 | 22 | 11 | 2 | 3 | 0 |

### Example 40

The Example was used to test the *in vivo* acute toxicity of the hyperbranched poly(amino acid)-based antimicrobial agents in animals, and *ε*-polylysine synthesized by fermentation was used as the control to evaluate the *in vivo* acute toxicity of the resulting hyperbranched poly(amino acid)-based antimicrobial agents in animals.

One hundred mice (Balb/C mice, purchased from Jilin University), half male and half female, weighing 21±3 g, were used. The hyperbranched poly(amino acid)-based antimicrobial agents prepared in Examples 23-37 were taken at a dose of 1 mg/mL respectively and intramuscularly injected into mice once a day for 15 consecutive days to observe the toxic reaction of mice. The experimental results showed that after intramuscular injection for 15 consecutive days, except for few mice with reduced vitality, the rest had no significant abnormal reactions, and all mice survived. It was demonstrated that the resulting hyperbranched poly(amino acid)-based antimicrobial agents have lower toxicity *in vivo.*

### Example 41

100 grams of ornithine was added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in diethyl ether, to give 82.7 grams of hyperbranched polyornithine.

### Example 42

2 g of hyperbranched polyornithine of Example 41 was dissolved under heating in 20 mL of N,N-dimethyl formamide (DMF), and 5 g of iodomethane was added. The reaction was conducted at 80 °C under stirring for 24 hours. After the stop of heating, it was cooled to room temperature, and precipitated in ethyl acetate, to give 2.4 g of quaternary ammonium salt-modified hyperbranched polyornithine.

### Example 43

91.32 grams of lysine hydrochloride and 20 grams of NaOH were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 6 hours. After the stop of heating, the polymer was dissolved with methanol and precipitated in diethyl ether, to give 72.8 grams of hyperbranched polylysine.

### Example 44

2 g of hyperbranched polylysine of Example 43 was dissolved in 5 mL of methanol, and acetyl chloride was slowly dropwise added at 0 °C. The system was warmed to room temperature and reacted for another 12 hours, then precipitated in ethyl acetate, to give 2.3 g of acetyl-modified hyperbranched polylysine.

### Example 45

2 g of hyperbranched polyornithine of Example 41 was dissolved in 5 mL of methanol, and 4.8 g of methylisothiourea hemisulphate and 5 mL of triethylamine were added. The reaction was conducted at 60 °C for 12 hours. After the stop of heating, it was cooled to room temperature and precipitated in ethyl acetate, to give 2.1 g of guanidino-modified hyperbranched polyornithine.

### Example 46

2 g of *ε-p*olylysine were dissolved into 5 mL of water, and 5.1 g of 1H-pyrazole-1-carboxamidine hydrochloride and 5 mL of triethylamine were added. The reaction was conducted at 60 °C for 12 hours. After the stop of heating, it was cooled to room temperature and precipitated in ethyl acetate, to give 2.2 g of guanidino-modified *ε-p*olylysine.

### Example 47

5.6 g of N-benzyloxycarbonyllysine was weighted into a 250 mL three necked flask, and 100 mL of tetrahydrofuran was added, stirred and dispersed. 2.5 g of triphosgene was carefully weighted and dissolved into 30 mL tetrahydrofuran, and slowly dropwise added to the reaction system under the protection of nitrogen. The system was refluxed under stirring for 3 hours until the solution was completely clear. After the reaction was completed, a large number of n-hexane was added such that the crude product was precipitated. The crude product was recrystallized twice using the tetrahydrofuran-n-hexane system, and dried under vacuum to give 4.96 g of product (yield: 88.6%). The NCA ring-opening of the lysine was initiated by using DMF as solvent and n-butylamine as initiator, to give *α*-polylysine.

### Example 48

2 g of *α*-polylysine of Example 47 were dissolved into 5 mL of water, and 5.1 g of 1H-pyrazole-1-carboxamidine hydrochloride and 5 mL of triethylamine were added. The reaction was conducted at 60 °C for 12 hours. After the stop of heating, it was cooled to room temperature and precipitated in ethyl acetate, to give 2.3 g of guanidino-modified *α*-polylysine.

### Example 49

80 grams of arginine and 20 grams of serine were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in diethyl ether, to give 81.2 grams of branched poly(amino acid).

### Example 50

2 g of poly(amino acid) of Example 49 was dissolved into 20 mL methanol and 0.5 g of p-toluenesulfonic acid was added, and the system was heated under reflux for 10 hours. The polymer was settled in diethyl ether, to give 2.2 g of ether group-modified poly(amino acid).

### Example 51

80 grams of arginine and 20 grams of glutamic acid were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in diethyl ether, to give 80.2 grams of branched poly(amino acid).

### Example 52

2 g of poly(amino acid) of Example 51 was dissolved in 20 mL of dry methanol, and 0.9 g of 1-(3-dimethylaminopropyl)-3-ethylcarbodiimide hydrochloride (EDCI) and 0.1 g of 4-imethylaminopyridine (DMAP) were added. The system was stirred at room temperature for 10 hours. The polymer was precipitated in diethyl ether, to give 2.1 g of methyl ester-modified poly(amino acid).

### Example 53

80 grams of ornithine and 20 grams of cysteine were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in diethyl ether, to give 80.2 grams of branched poly(amino acid).

### Example 54

2 g of poly(amino acid) of Example 53 was dissolved in 20 mL of dry DMF. The argon gas was introduced for 30 min to remove the oxygen. 0.9 g of propynol and 0.1 g DMAP were added. The system was stirred at room temperature for 10 min. The reaction mixture was reacted at room temperature under the irradiation of ultraviolet light (365nm) for 120 min. Then, the polymer was precipitated in diethyl ether, to give 2.1 g of hydroxyl-modified poly(amino acid).

### Example 55

80 grams of histidine and 20 g of serine were added to a 500 mL round bottom flask, and a water separator was connected. The nitrogen purge was conducted for three times (each for more than 10 minutes) so as to finally maintain nitrogen atmosphere. The reaction was conducted at 180 °C under stirring and heating for 4 hours. After the stop of heating, the reaction system was cooled to room temperature. The polymer was dissolved with methanol and precipitated in diethyl ether, to give 80.2 grams of branched poly(amino acid).

### Example 56

2 g of poly(amino acid) of Example 55 was dissolved into 20 mL methanol and 0.5 g of p-toluenesulfonic acid was added, and the system was heated under reflux for 10 hours. The polymer was precipitated in diethyl ether, to give 2.2 g of ether group-modified poly(amino acid).

### Example 57

36 mg of poly(amino acid)s prepared in Examples 41-56 were respectively weighted and dissolved into 3 mL of sterile PBS, to give 12 mg/mL of stock solution. The antimicrobial activity of the poly(amino acid)-based antimicrobial agents was measured in accordance with the following method, and a part of experiment results were shown in Tables 5-1 and 5-2.

The various strains used in the following examples were purchased from the National Institute for the Control of Biological Products.

The antimicrobial activity of the hyperbranched poly(amino acid)-based antimicrobial agents was tested through the 96-well plate method, and *ε*-polylysine (*M*ₙ=4000 g/mol) synthesized by fermentation was used as the control to evaluate the antimicrobial capacity of the resulting poly(amino acid)-based antimicrobial agents. The minimum inhibitory concentration (MIC) is defined as the lowest polymer concentration that inhibits microbial growth by 90% compared to the control group.

A small amount of strains were picked from the agar slant medium with the inoculating ring to the ordinary M-H medium, incubated at 37°C overnight to recover the strains and achieve exponential growth. The microbial solution was diluted so that the concentration of the microbial solution was 10⁶ CFU/mL. In each well, 175 µL of microbial solution and 25 µL of polymer solutions at different concentrations were added. The 96-well plate was incubated at 37°C for 20 hours, and the OD₆₀₀ value was measured by the microplate reader.

**Table 5-1. Comparisons of MIC values of different antimicrobial polymers against different microbes**

| Strain Name | | MIC values of different antimicrobial polymers against different microbes(µg/mL) | | | | | | |
|---|---|---|---|---|---|---|---|---|
| | | Ex. 41 | Ex. 42 | Ex. 43 | Ex. 44 | Ex. 45 | Ex. 46 | ε-polylysine |
| Gram-negative bacteria | *Escherichia coli* A TCC8739 | 47 | 24 | 47 | 24 | 12 | 24 | 47 |
| | *Pseudomonas aeruginosa* A TCC9207 | 47 | 24 | 47 | 24 | 12 | 12 | 47 |
| | *Klebsiella pneumoniae* A TCC700603 | 47 | 24 | 96 | 47 | 47 | 24 | 94 |
| | *Salmonella paratyphi B* CMCC50094 | 24 | 12 | 24 | 12 | 24 | 6 | 47 |
| | *Acinetobacter baumannii* ATCC 19606 | 24 | 12 | 24 | 12 | 12 | 12 | 24 |
| Gram-positive bacteria | *Staphylococcus aureus* ATCC 25923 | 24 | 12 | 24 | 12 | 12 | 12 | 24 |
| | Methicillin-resistant *Staphylococcus aureus* ATCC 43300 | 24 | 6 | 24 | 24 | 6 | 12 | 47 |
| | *Bacillus subtilis* ATCC 6633 | 24 | 6 | 24 | 12 | 12 | 12 | 24 |
| | *Micrococcus luteus* ATCC 10240 | 47 | 12 | 24 | 24 | 12 | 6 | 24 |
| | *Bacillus pumilus* ATCC700814 | 12 | 6 | 47 | 12 | 12 | 12 | 24 |
| Fungi | *Candida albicans* ATCC10231 | 24 | 12 | 24 | 12 | 12 | 12 | 47 |
| | *Saccharomyces cerevisiae* ATCC 9763 | 24 | 12 | 24 | 12 | 12 | 24 | 47 |

**Table 5-2. Comparisons of MIC values of different antimicrobial polymers against different microbes**

| Strain Name | | MIC values of different antimicrobial polymers against different microbes(µg/mL) | | | | | | |
|---|---|---|---|---|---|---|---|---|
| | | Ex. 47 | Ex. 48 | Ex. 50 | Ex. 52 | Ex. 54 | ε-polylysine | α-polylysine |
| Gram-negative bacteria | *Escherichia coli* ATCC8739 | 47 | 24 | 24 | 24 | 12 | 47 | 47 |
| | *Pseudomonas aeruginosa* A TCC9207 | 94 | 12 | 24 | 12 | 12 | 47 | 94 |
| | *Klebsiella pneumoniae* A TCC700603 | 94 | 12 | 24 | 24 | 12 | 94 | 94 |
| | *Salmonella paratyphi B* CMCC50094 | 47 | 24 | 24 | 12 | 24 | 47 | 47 |
| | *Acinetobacter baumannii* ATCC 19606 | 47 | 24 | 47 | 12 | 12 | 24 | 47 |
| Gram-positive bacteria | *Staphylococcus aureus* ATCC 25923 | 94 | 24 | 12 | 24 | 6 | 24 | 94 |
| | Methicillin-resistant *Staphylococcus aureus* ATCC 43300 | 94 | 12 | 24 | 47 | 12 | 47 | 94 |
| | *Bacillus subtilis* ATCC 6633 | 24 | 6 | 6 | 24 | 6 | 24 | 24 |
| | *Micrococcus luteus* ATCC 10240 | 47 | 24 | 12 | 24 | 6 | 24 | 48 |
| | *Bacillus pumilus* ATCC700814 | 24 | 12 | 6 | 12 | 12 | 24 | 24 |
| Fungi | *Candida albicans* ATCC10231 | 24 | 12 | 24 | 12 | 12 | 47 | 24 |
| | *Saccharomyces cerevisiae* ATCC 9763 | 47 | 24 | 24 | 12 | 12 | 47 | 47 |

### Example 58

36 mg of poly(amino acid)s prepared in Examples 41-56 were weighted respectively and dissolved into 3 mL of sterile PBS, to give 12 mg/mL of stock solution. The *in vitro* hemolytic activity of the poly(amino acid)-based antimicrobial agents was measured in accordance with the following method, and a part of experiment results were shown in Tables 6-1 and 6-2.

The *in vitro* hemolytic activity of the poly(amino acid)-based antimicrobial agents was tested by a 96-well plate method, and *ε*-polylysine synthesized by fermentation was used as the control to evaluate the *in vitro* hemolytic activity of the resulting poly(amino acid)-based antimicrobial agents.

Preparation of 2% (v/v) erythrocyte suspension: 2 mL of fresh healthy human blood was taken and dilute with 10 mL of endotoxin-free PBS buffer solution. The blood was transferred to a triangular flask with glass beads and shaken for 10 minutes, or stirred with a glass rod, to remove the fibrin and make it into deflbrinaled blood. The deflbrinated blood was centrifuged at 20 °C at 1000∼1500 r/min for 10∼15 minutes, and the supernatant was removed. The precipitated erythrocyte was then washed with the PBS buffer solution for 4 times in accordance with the above method, until the supernatant does not appear red. The obtained erythrocyte was formulated into a 2% suspension with physiological saline for subsequent experiments.

The polymer was diluted with the PBS buffer solution to prepare solutions of different concentrations and added to 96-well plates. The PBS buffer solution alone was used as the negative control, and 0.2% Triton-X-100 dissolved in water was used as the positive control for *in vitro* hemolytic activity test. The washed erythrocyte (2% v/v, 50 µL) was added to the 96-well plate, mixed well and incubated. The absorption at 540 nm was measured with a microplate reader.

**Table 6-1. Test results for the in vitro hemolytic activities of different antimicrobial polymers**

| Concentration of the antimicrobial polymer (µg/mL) | Hemolysis ratio (%) | | | | | | |
|---|---|---|---|---|---|---|---|
| | Ex. 41 | Ex. 42 | Ex. 43 | Ex. 44 | Ex. 45 | *ε-*polylysine | α-polylysine |
| 3 | 0 | 0 | 0 | 0 | 0 | 0 | 0 |
| 6 | 0 | 0 | 0 | 0 | 0 | 0 | 0 |
| 12 | 0 | 0 | 0 | 0 | 0 | 0 | 0 |
| 24 | 0 | 0 | 0 | 0 | 0 | 0 | 0 |
| 47 | 0 | 0 | 0 | 0 | 0 | 0 | 0 |
| 94 | 0 | 0 | 0 | 0 | 0 | 0 | 0 |
| 188 | 0 | 0 | 0 | 0 | 0 | 0 | 0 |
| 375 | 0 | 1 | 0 | 0 | 0 | 0 | 1 |
| 750 | 1 | 4 | 1 | 0 | 1 | 2 | 3 |
| 1500 | 3 | 6 | 2 | 1 | 2 | 3 | 9 |

**Table 6-2. Test results for the in vitro hemolytic activities of different antimicrobial polymers**

| Concentrati on of the antimicrobi al polymer (µg/mL) | Hemolysis ratio (%) | | | | | | |
|---|---|---|---|---|---|---|---|
| | Ex. 46 | Ex. 48 | Ex. 50 | Ex. 52 | Ex. 54 | *ε-*polylysine | α-polylysine |
| 3 | 0 | 0 | 0 | 0 | 0 | 0 | 0 |
| 6 | 0 | 0 | 0 | 0 | 0 | 0 | 0 |
| 12 | 0 | 0 | 0 | 0 | 0 | 0 | 0 |
| 24 | 0 | 0 | 0 | 0 | 0 | 0 | 0 |
| 47 | 0 | 0 | 0 | 0 | 5 | 0 | 0 |
| 94 | 0 | 0 | 0 | 0 | 8 | 0 | 0 |
| 188 | 0 | 0 | 0 | 0 | 12 | 0 | 0 |
| 375 | 0 | 0 | 0 | 0 | 19 | 0 | 1 |
| 750 | 0 | 2 | 0 | 1 | 32 | 2 | 3 |
| 1500 | 1 | 4 | 0 | 1 | 54 | 3 | 9 |

### Example 59

The Example was used to test the *in vivo* acute toxicity of the poly(amino acid)-based antimicrobial agents in animals, and *ε*-polylysine synthesized by fermentation was used as the control to evaluate the *in vivo* acute toxicity of the resulting poly(amino acid)-based antimicrobial agents in animals.

One hundred mice (Balb/C mice, purchased from Jilin University), half male and half female, weighing 21±3 g, were used. The poly(amino acid)-based antimicrobial agents prepared in Examples 41-56 were taken at a dose of 1 mg/mL respectively and intramuscularly injected into mice once a day for 15 consecutive days to observe the toxic reaction of mice. The experimental results showed that after intramuscular injection for 15 consecutive days, except for few mice with reduced vitality, the rest had no significant abnormal reactions, and all mice survived. It was demonstrated that the resulting poly(amino acid)-based antimicrobial agents have lower toxicity *in vivo.*

### Example 60

50 mL of styrene, 50 mL of chloromethylstyrene, and azodiisobutyronitrile (both styrene and chloromethylstyrene passed through a section of neutral alumina column to remove the polymerization inhibitor) were added to a three-necked bottle equipped with the nitrogen port, stirrer, and thermometer, stirred, and N₂ was bubbled for 30 min. The nitrogen atmosphere was maintained, and the reaction was conducted at 70 °C for 24 hours. After the reaction was completed, the system was precipitated in ethanol, to give chloromethylated polystyrene.

5 g of chloromethylated polystyrene, 0.5 g of hyperbranched polylysine prepared in Example 14, and 30 mL of distilled water were added to the reaction bottle. After bubbling N₂ for 30 min, the bottle was sealed and the reaction was conducted at 120 °C under nitrogen atmosphere for 10 hours. The solid product was filtered and rinsed with a large amount of distilled water, to give hyperbranched polylysine-modified polystyrene (HBPL-PS). The HBPL-PS was pressed at high temperature into a sample film of 2 cm × 1.5 cm for antimicrobial test.

By using *Escherichia coli* and *Staphylococcus aureus,* we studied the antimicrobial adhesion performances of the material. First, *Escherichia coli* (ATCC8739) and *Staphylococcus aureus* (ATCC25923) were cultured in LB and TSB medium at 37 °C for 24 hours, respectively. Then, the medium containing bacteria was centrifuged at 2700 rmp for 10 min. The supernatant was removed, and resuspended to a concentration of 1 × 10⁸ cells/mL. The sample membrane was transferred to a 48-well plate, and 1 mL of bacterial suspension was added respectively to adhere for 1 h and then the medium was aspirated. After that, *Escherichia coli* (using 0.5% (wt/vol) glucose medium) and *Staphylococcus aureus* (using TSB medium) was continued with static culture at 37 °C for 4 hours. The sample film adhered with bacteria was put in 1 mL of fresh sterile PBS solution and ultrasonically cleaned for 1 min to achieve the desorption of bacteria from the surface. The PBS solution containing bacteria was diluted by a certain fold, and applied to the solid medium. After culturing at 37 °C overnight, the bacterial colonies formed on the surface of the solid medium was counted. Compared with polystyrene (PS) as the control group, the bacterial adhesion of *Escherichia coli* and *Staphylococcus aureus* on the surface of HBPL-PS decreased by ∼95.5% and ∼98.8%, respectively.

## Claims

1. A branched poly(amino acid) antimicrobial agent, comprising a branched poly(amino acid);
the branched poly(amino acid) is obtained by the homopolymerization of one amino acid unit, or by the copolymerization of two or more amino acid units;
the amino acid unit has a structure of Formula I or salts thereof: 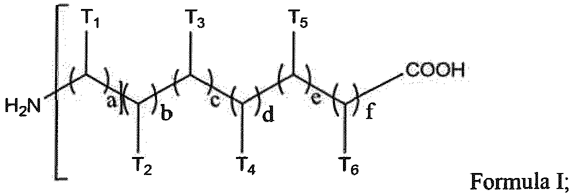 wherein,
a, b, c, d, e, and f are independently an integer of 0-6, and 1≤a+b+c+d+e+f≤20, preferably a+b+c+d+e+f≤10;
T₁, T₂, T₃, T₄, T₅, and T₆ are independently selected from the group consisting of hydrogen, hydroxyl, mercapto, amino, carboxyl, C1∼C18 alkyl and derivatives thereof, C6∼ C30 aryl and derivatives thereof, C3∼C8 cycloalkyl and derivatives thereof, C2∼C8 alkenyl and derivatives thereof, C2∼C8 alkynyl and derivatives thereof, C1∼C8 alkoxy and derivatives thereof, C1∼C8 alkylthio and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof.

2. The antimicrobial agent according to claim 1, wherein the branched poly(amino acid) is obtained by the homopolymerization of one amino acid unit, wherein at least one of T₁, T₂, T₃, T₄, T₅, and T₆ of the amino acid unit is selected from the group consisting of hydroxyl, amino, mercapto, carboxyl, C2∼C8 alkenyl and derivatives thereof, C2∼C8 alkynyl and derivatives thereof, C1∼C8 alkoxy and derivatives thereof, C1∼C8 alkylthio and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof.

3. The antimicrobial agent according to claim 1, wherein the branched poly(amino acid) is obtained by the copolymerization of two or more amino acid units, wherein at least one of T₁, T₂, T₃, T₄, T₅, and T₆ of at least one amino acid unit is selected from the group consisting of hydroxyl, amino, mercapto, carboxyl, C2∼C8 alkenyl and derivatives thereof, C2∼C8 alkynyl and derivatives thereof, C1∼C8 alkoxy and derivatives thereof, C1∼C8 alkylthio and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof.

4. The antimicrobial agent according to any one of claims 1-3, wherein T₁, T₂, T₃, T₄, T₅, and T₆ are independently selected from the group consisting of any one of the following structures: 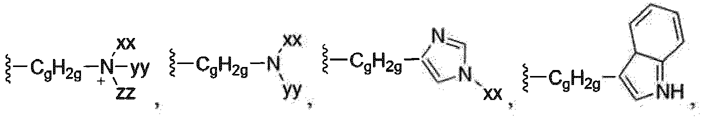 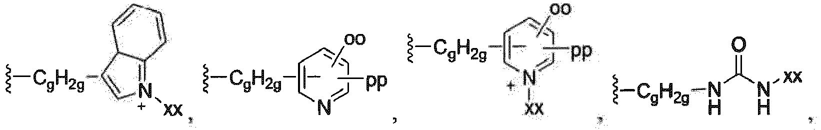 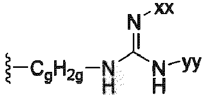 or salts thereof, 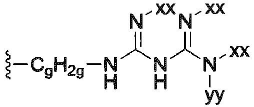 or salts thereof, 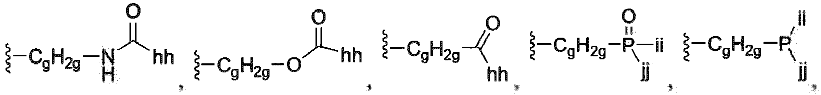 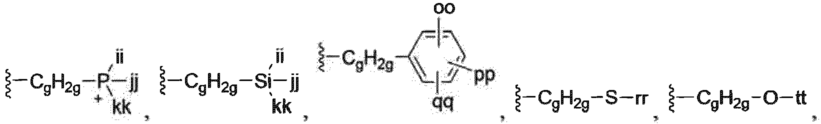 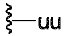 wherein, g is an integer of 0 to 10; wherein, xx, yy, and zz are independently selected from the group consisting of hydrogen, C1∼C18 alkyl, C6∼C30 aryl, C3∼C18 cycloalkyl, carbonyl derivatives; hh is independently selected from the group consisting of hydrogen, hydroxyl, amino, halogen elements, C1∼C18 alkyl, C6∼C30 aryl, C3∼C18 cycloalkyl, amine and derivatives thereof, alkoxy derivatives, alkylthio derivatives; ii, jj, and kk are independently selected from the group consisting of hydrogen, C1∼C18 alkyl, C6∼C30 aryl, C3∼C18 cycloalkyl, alkoxy and derivatives thereof; oo, pp, and qq are independently selected from the group consisting of hydrogen, carboxyl, hydroxyl, amino, C1∼C18 alkyl, C6∼C30 aryl, C3∼C18 cycloalkyl, halogens, amine and derivatives thereof, alkoxy derivatives, carbonyl derivatives; rr and tt are independently selected from the group consisting of hydrogen, C1∼C18 alkyl, C6∼C30 aryl, C3∼C18 cycloalkyl, alkylthio derivatives, alkoxy derivatives, carbonyl derivatives; and uu is independently selected from the group consisting of one or more of the structures represented by the following formulae: CₙH₂ₙ₊₁₋ₕTₕ (n is an integer of 0 to 10), CₙH₂ₙ₋₁₋ₕTₕ (n is an integer of 2 to 10), CₙH₂ₙ₋₃₋ₕTₕ (n is an integer of 2 to 10), CₙH₂ₙ₋₇₋ₕTₕ (n is an integer of 6 to 18), wherein, h is an integer of 0 to 3, and T is independently selected from any one or more of halogen elements.

5. The antimicrobial agent according to any one of claims 1-4, wherein T₁, T₂, T₃, T₄, T₅, and T₆ are independently selected from the group consisting of any one of the following structures:

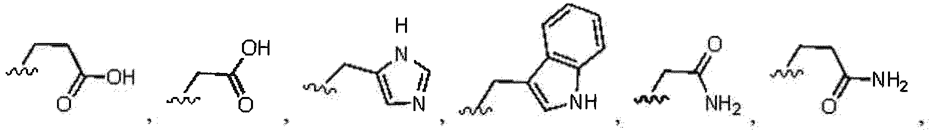

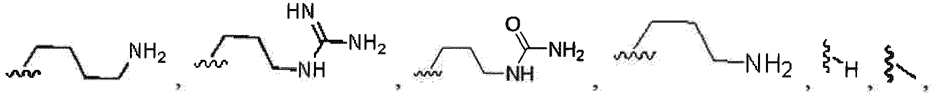

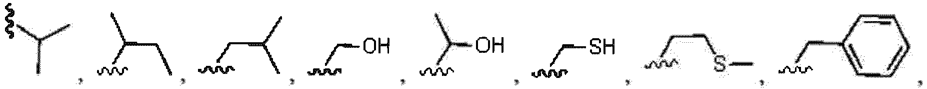

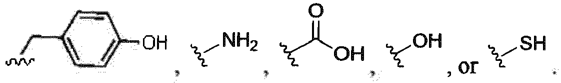

6. The antimicrobial agent according to any one of claims 1, 2, 4, and 5, wherein the branched poly(amino acid) is obtained by the homopolymerization of one amino acid unit, and the amino acid has a functionality of ≥3.

7. The antimicrobial agent according to claim 6, wherein the amino acid unit is glutamic acid, lysine, arginine, ornithine, histidine, aspartic acid, tryptophan, serine, citrulline, tyrosine, cysteine, asparagine, glutamine, or threonine, preferably the amino acid unit is lysine, arginine, ornithine, or histidine.

8. The antimicrobial agent according to any one of claims 1, 3, 4, and 5, wherein the copolymerization unit of the branched poly(amino acid) contains at least one or more amino acid having a functionality of ≥3, wherein the amino acid unit having a functionality of ≥3 accounts for δ of total amino acid units, 0<δ≤100%.

9. The antimicrobial agent according to claim 8, wherein the amino acid unit having a functionality of ≥3 is one or more of glutamic acid, lysine, ornithine, arginine, histidine, asparagine, glutamine,serine, tryptophan, aspartic acid, citrulline, threonine, tyrosine, and cysteine; preferably the amino acid unit includes at least one or more of lysine, ornithine, arginine, and histidine.

10. The antimicrobial agent according to claim 1, wherein the poly(amino acid) is subjected to any one or more of the following modifications:
I. modifying the amino or the amino group in the amides into the following groups: 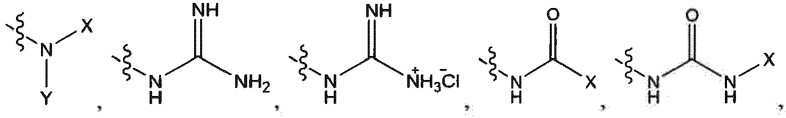 or 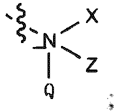
II. modifying the hydroxyl group into -OR₁ or -OC(=O)R₂,
III. modifying the mercapto group into -SR₃;
IV. modifying the carboxyl group into -C(=O)NHR₄ or -C(=O)OR₅,
V. modifying the guanidine group into the group as shown by Formula V-1;
VI. modifying the NH in the nitrogen-containing heterocyclyl into NR₆; 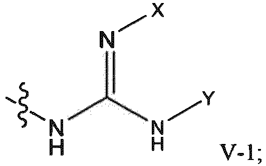
wherein, X, Y, Z, and Q are independently selected from the group consisting of hydrogen. C1∼C18 alkyl and derivatives thereof, C6∼C30 aryl and derivatives thereof, C3∼C18 cycloalkyl and derivatives thereof, C2∼C18 alkenyl and derivatives thereof, C2∼C18 alkynyl and derivatives thereof, C1∼C18 alkoxy and derivatives thereof, carboxylic acid and derivatives thereof, amine and derivatives thereof, nitrogen-containing heterocyclic group and derivatives thereof, oxygen-containing heterocyclic group and derivatives thereof, or sulfur-containing heterocyclic group and derivatives thereof;
R₁, R₂, R₃, R₄, R₅, and R₆ are independently selected from the group consisting of H, C1∼C18 alkyl and derivatives thereof, C6∼C30 aryl and derivatives thereof, C3∼C18 cycloalkyl and derivatives thereof, C2∼C18 alkenyl and derivatives thereof, C2∼C18 alkynyl and derivatives thereof, C1∼C18 alkoxy and derivatives thereof, carboxylic acid and derivatives thereof, amino and derivatives thereof, nitrogen-containing heterocyclic group and derivatives thereof, oxygen-containing heterocyclic group and derivatives thereof, or sulfur-containing heterocyclic group and derivatives thereof; and R₁, R₃, R₅, and R₆ are not H.

11. The antimicrobial agent according to claim 10, wherein X, Y, Z, and Q are independently selected from the group consisting of hydrogen, C1∼C3 alkyl, C6∼C8 aryl, C3∼C6 cycloalkyl, C1∼C3 alkoxy, C2∼C5 nitrogen-containing heterocyclic group, C2∼C5 oxygen-containing heterocyclic group, or C2∼C5 sulfur-containing heterocyclic group; and
R₁, R₂, and R₃ are independently selected from the group consisting of C1∼C3 alkyl, C6∼C8 aryl, C3∼C6 cycloalkyl, C1∼C3 alkoxy, C2∼C5 nitrogen-containing heterocyclic group, C2∼C5 oxygen-containing heterocyclic group, or C2∼C5 sulfur-containing heterocyclic group.

12. The antimicrobial agent according to any one of claims 1-11, wherein the branched poly(amino acid) has a number-average molecular weight in the range of 500 g/mol-500,000 g/mol.

13. The antimicrobial agent according to claim 1, further comprising an adjuvant.

14. The antimicrobial agent according to claim 1, wherein the antimicrobial agent is used in one or more forms selected from the group consisting of solid, solution, suspension, emulsion, hydrogel, oleogel, or aerosol, being coated or grafted onto the solid surface, or being blended with other materials.

15. The antimicrobial agent according to any one of claims 1-14, for use in one or more of bacteria, virus, fungus, actinomyces, rickettsia, mycoplasma, chlamydia, and spirochete.
